# EUROPEAN PATENT SPECIFICATION

(11) **EP 3 116 376 B1**
(45) Date of publication and mention of the grant of the patent: **11.08.2021**
(21) Application number: 14885751.9
(22) Date of filing: 15.09.2014
(51) Int. Cl.: A61B 90/00, A61B 1/00, A61B 6/02, A61B 34/20, A61B 34/10

(54) **METHODS AND SYSTEMS FOR INTRAOPERATIVELY CONFIRMING LOCATION OF TISSUE STRUCTURES**
VERFAHREN UND SYSTEME ZUR INTEROPERATIVEN BESTÄTIGUNG DER STELLE VON GEWEBESTRUKTUREN
PROCÉDÉS ET SYSTÈMES DE CONFIRMATION PEROPÉRATOIRE D'EMPLACEMENT DE STRUCTURES DE TISSU

(30) Priority: 14.03.2014 WO PCT/CA2014/050268
(43) Date of publication of application: 18.01.2017
(73) Proprietor: Synaptive Medical Inc., Toronto, ON M5V 3B1 (CA)
(72) Inventor: WOOD, Michael, Toronto, Ontario M6H 2P4 (CA); PIRON, Cameron, Toronto, Ontario M5R 2E4 (CA); YUWARAJ, Murugathas, Markham, Ontario L6C 2P3 (CA); SELA, Gal, Toronto, Ontario M4W 2X5 (CA); RICHMOND, Joshua, Toronto, Ontario M3H 2G6 (CA); MCFADYEN, Stephen, Toronto, Ontario M6J 0A4 (CA)
(74) Representative: Köllner, Malte
(86) International application number: PCT/CA2014/050877
(87) International publication number: WO 2015/135058

(56) References cited:
- WO-A1-2013/116305
- WO-A2-2008/111070
- WO-A2-2012/058217
- US-B2- 7 418 169
- US-B2- 7 538 859
- US-B2- 8 862 206

## Description

### FIELD

The present disclosure relates to imaging methods for use in minimally invasive therapy and image guided medical procedures using optical imaging, and more particularly, hyperspectral imaging.

### BACKGROUND

The optical absorption and scattering properties of biological tissue depend on both the chemical and structural properties of the tissue and the wavelength of the interacting light. How these absorption and scattering properties of tissue change as a function of light can be particularly useful, as it is often unique to chemicals or structures in the tissue (the spectrum of the tissue). For example the absorption features of oxy- and deoxy-hemoglobin can be used to measure the oxygenation of blood and tissue, and the scatter changes caused by difference cellular sizes can be used to detect precancerous and cancerous tissue. The field of measuring these changes in optical properties, as a function of light, is known as spectroscopy and the device to measure the light at the various wavelengths is known as a spectrometer. Spectroscopy has found a wealth of current and potential applications in medicine.

Traditional spectrometers measure the spectrum of light from a single point of a sample. However, the spectrum from multiple spatial points can be combined to form a 3D spatial dataset (sometimes referred to as a hypercube), where the first two dimensions are spatial and the third is wavelength. In other words, each image pixel has an entire spectrum rather than just an intensity or RBG value. This is known as hyperspectral imaging and is a powerful technique as spatially resolved tissue chemical or microstructural properties can imaged, thus providing a more complete understanding of the tissue and may be a useful technique for tissue differentiation. According to a paper by Dicker et al [Differentiation of Normal Skin and Melanoma using High Resolution Hyperspectral Imaging], hyperspectral image analysis (or hyperspectral imaging) was applied to search for spectral differences between benign and malignant dermal tissue in routine hematoxylin eosin stained specimens (i.e., normal and abnormal skin, benign nevi and melanomas). The results revealed that all skin conditions in the initial data sets could be objectively differentiated providing that staining and section thickness was controlled.

WO 2008/111070 discloses a method and systems for performing medical procedures in tree-like luminal structures within a subject's body using an image-guided navigation system and various types of probes including ultrasound probes, MRI probes and optical coherence tomography probes.

WO 2012/058217 discloses a non-invasive imaging system for imaging patients which includes a storage system for storing the imaging results and also includes pre-stored medical images on the site being imaged and an atlas containing structural information of the site being imaged.

### SUMMARY

The present invention is defined by the independent claim. Preferred embodiments are given in the dependent claims. Systems, methods and devices are provided for intraoperatively confirming location of tissue structures during medical procedures. In an embodiment, preoperative image data of a patient's skeletal structure in a vicinity of an anatomical part undergoing a medical procedure is acquired. During the procedure, after exposing tissue intraoperative image data is acquired by scanning a selected region of tissue, in a vicinity of the skeletal structure using Polarization Sensitive-Optical Coherence Tomography (PS-OCT). Regions of tissue exhibiting structural organization in the vicinity of the skeletal structure are identified from the intraoperative (PS-OCT) image data. Geometrically correlating and registering the intraoperative (PS-OCT) image data with the preoperative image data of the skeletal structure in the vicinity of the anatomical part is then performed using a priori known anatomical information about the regions of tissue exhibiting structural information.

In another embodiment, global preoperative image data of tissue in an anatomical part is acquired using contrast based magnetic resonance imaging and identifying. From the image data, a global vascular structure within the tissue is identified. After exposing tissue during a medical procedure in the anatomical part, intraoperative image data is acquired by scanning, using hyperspectral imaging, of a selected local region of the tissue. From this intraoperative hyperspectral image data, a local vascular structure in the selected local region of the tissue is located and identified. The global vascular image data is searched for identifying and locating a portion of the global vascular structure geometrically matching the local vascular structure. Upon identifying and locating matching vascular structure between the two imaging modalities, one or more local vascular structures in the selected local region of the tissue is registered with the global vascular structure within the tissue for confirming location of the one or more local vasculature structures.

Thus, there is disclosed herein a computer implemented method of intraoperatively confirming location of organized tissue structures in relation to a patient's skeletal structure during a medical procedure, comprising:
acquiring preoperative image data of a patient's skeletal structure in a vicinity of an anatomical part undergoing a medical procedure;
after exposing tissue during a medical procedure in the anatomical part, acquiring intraoperative image data by scanning a selected region of tissue, in a vicinity of the skeletal structure in the anatomical part undergoing the medical procedure using Polarization Sensitive-Optical Coherence Tomography (PS-OCT);
identifying, from the intraoperative (PS-OCT) image data, regions of tissue exhibiting structural organization in the vicinity of the skeletal structure; and
using a priori known anatomical information about the regions of tissue exhibiting structural information for geometrically correlating and registering the intraoperative (PS-OCT) image data with the preoperative image data of the skeletal structure in the vicinity of the anatomical part.

The pre-operative image data of the skeletal structure in a vicinity of an anatomical part undergoing a medical procedure may be acquired using any one of computed tomography (CT), magnetic resonance imaging (MRI) and optical coherence tomography (OCT).

An example of the MRI technique is T1 magnetic resonance imaging (T1 MRI).

The tissue exhibiting structural organization includes, ligaments, tendons, muscle, cartilage, connective membranes, nerves, retina, blood vessel walls, some bone structures, trachea, esophagus, tongue, teeth and other connective tissues.

The a priori known anatomical information about the regions of tissue exhibiting structural information may include attachment points of tissue exhibiting structural information to the skeletal structure relative to landmark positions on the skeletal structure.

Disclosed herein is a method, comprising the steps of:
a) intraoperatively confirming location of organized tissue structures in relation to a patient's skeletal structure during a medical procedure, by:
   acquiring preoperative image data of a patient's skeletal structure in a vicinity of an anatomical part undergoing a medical procedure;
   after exposing tissue during a medical procedure in the anatomical part, acquiring intraoperative image data by scanning a selected region of tissue, in a vicinity of the skeletal structure in the anatomical part undergoing the medical procedure using Polarization Sensitive-Optical Coherence Tomography (PS-OCT);
   identifying, from the intraoperative (PS-OCT) image data, regions of tissue exhibiting structural organization in the vicinity of the skeletal structure; and
   using a priori known anatomical information about the regions of tissue exhibiting structural information for geometrically correlating and registering the intraoperative (PS-OCT) image data with the preoperative image data of the skeletal structure in the vicinity of the anatomical part; and
b) using the registered intraoperative (PS-OCT) image data with the preoperative image data of the skeletal structure in the vicinity of the anatomical part to plan a surgical trajectory to avoid selected regions of the tissue exhibiting structural information.

Also disclosed is a computer implemented system for intraoperatively confirming location of organized tissue structures in relation to a patient's skeletal structure during a medical procedure, comprising:
a Polarization Sensitive-Optical Coherence Tomography (PS-OCT) apparatus configured to scan a selected region of tissue after the tissue is exposed during the medical procedure to acquire intraoperative image data of the selected region of tissue;
a computer processor having a memory storage, said Polarization Sensitive-Optical Coherence Tomography being connected to the computer processor, said memory storage having stored therein preoperative image data of a patient's skeletal structure in a vicinity of an anatomical part undergoing a medical procedure, said memory storage having stored therein a priori known anatomical information about the regions of tissue exhibiting structural information;
said computer processor being programmed with instructions to
   a) identify, from the intraoperative (PS-OCT) image data, regions of tissue exhibiting structural organization in the vicinity of the skeletal structure; and
   b) use the stored priori known anatomical information about the regions of tissue exhibiting structural information to geometrically correlate and register the intraoperative (PS-OCT) image data with the preoperative image data of the skeletal structure in the vicinity of the anatomical part.

The present disclosure also provides a computer implemented method of intraoperatively confirming location of vasculature structures located below a surface tissue during a medical procedure, comprising:
acquiring global preoperative image data of tissue in anatomical part undergoing a medical procedure using contrast based magnetic resonance imaging and identifying, from the image data, a global vascular structure within the tissue;
after exposing tissue during a medical procedure in the anatomical part, acquiring intraoperative image data by scanning, using hyperspectral imaging, a selected local region of the tissue in the anatomical part undergoing the medical procedure;
identifying, from the intraoperative hyperspectral image data, a local vascular structure in the selected local region of the tissue; and
searching the global vascular image data for identifying and locating a portion of the global vascular structure geometrically matching the local vascular structure, and upon identifying and locating matching vascular structure, geometrically correlating and registering the local vascular structure in the selected local region of the tissue with the a global vascular structure within the tissue for confirming location of the local vasculature structures.

A method is disclosed, comprising the steps of:
acquiring global preoperative image data of tissue in anatomical part undergoing a medical procedure using contrast based magnetic resonance imaging and identifying, from the image data, a global vascular structure within the tissue;
after exposing tissue during a medical procedure in the anatomical part, acquiring intraoperative image data by scanning, using hyperspectral imaging, a selected local region of the tissue in the anatomical part undergoing the medical procedure;
identifying, from the intraoperative hyperspectral image data, a local vascular structure in the selected local region of the tissue;
searching the global vascular image data for identifying and locating a portion of the global vascular structure geometrically matching the local vascular structure, and upon identifying and locating matching vascular structure, geometrically correlating and registering the local vascular structure in the selected local region of the tissue with the a global vascular structure within the tissue for confirming location of the local vasculature structures; and
b) using the registered hyperspectral image data with the preoperative image data of the vascular structure in the tissue of the anatomical part to plan a surgical trajectory to navigate through selected regions of the tissue exhibiting vascular structure.

A further understanding of the functional and advantageous aspects of the disclosure can be realized by reference to the following detailed description and drawings.

### BRIEF DESCRIPTION OF THE DRAWINGS

Embodiments will now be described, by way of example only, with reference to the drawings, in which:
**FIG. 1** shows an example navigation system to support minimally invasive access port-based surgery.
**FIG. 2** is block diagram illustrating system components of a navigation system.
**FIG. 3** is a flow chart illustrating the processing steps involved in a port-based surgical procedure using a navigation system.
**FIG. 4** is an example embodiment port based brain surgery using a video scope.
**FIG. 5A** is an example embodiment of a video scope with camera coupler and illumination optics.
**FIG. 5B** is an example embodiment of a fiber bundle used to deliver light from external light source to the video scope.
**FIG. 5C** is an example embodiment of a video scope and illumination assembly.
**FIG. 6** illustrates an example imaging optical sub-system of the video scope.
**FIG. 7** illustrates the arrangement of illumination optics and filter wheel for wide field of view arrangement.
**FIG. 8A**illustrates the non-uniform illumination obtained at the distal end of port with two illumination sources and a port with reflective surface.
**FIG. 8B** illustrates the near-uniform illumination obtained at the distal end of the port with two illumination sources and a port with rough surface.
**FIG. 9** an example embodiment illustrating a standard hyperspectral imaging system.
**FIG. 10** is a flow chart illustrating a method to acquire hyperspectral data and white-light images in a multiplex fashion.
**FIG. 11** is an example embodiment illustrating imaging at specific wavelength bands.
**FIG. 12** shows an example, non-limiting implementation of computer control system.

### DETAILED DESCRIPTION

Various embodiments and aspects of the disclosure will be described with reference to details discussed below. The following description and drawings are illustrative of the disclosure and are not to be construed as limiting the disclosure. Numerous specific details are described to provide a thorough understanding of various embodiments of the present disclosure. However, in certain instances, well-known or conventional details are not described in order to provide a concise discussion of embodiments of the present disclosure.

As used herein, the terms, "comprises" and "comprising" are to be construed as being inclusive and open ended, and not exclusive. Specifically, when used in the specification and claims, the terms, "comprises" and "comprising" and variations thereof mean the specified features, steps or components are included. These terms are not to be interpreted to exclude the presence of other features, steps or components.

As used herein, the term "exemplary" means "serving as an example, instance, or illustration," and should not be construed as preferred or advantageous over other configurations disclosed herein.

As used herein, the terms "about" and "approximately" are meant to cover variations that may exist in the upper and lower limits of the ranges of values, such as variations in properties, parameters, and dimensions. In one non-limiting example, the terms "about" and "approximately" mean plus or minus 10 percent or less.

Port-based surgery is a minimally invasive surgical technique where a port is introduced to access the surgical region of interest using surgical tools. Unlike other minimally invasive techniques, such as laparoscopic techniques, the port diameter is larger than tool diameter. Hence, the tissue region of interest is visible through the port. Accordingly, exposed tissue in a region of interest at a depth few centimetres below the skin surface, and accessible through a narrow corridor in the port, may be visualized using externally positioned optical systems such as microscopes and video scopes.

Current methods of tissue differentiation during port-based surgical procedure involves visual verification using externally placed video scope. Tissue differentiation may be useful because surgeons do not have a quantitative means of effectively confirming tissues types during a surgical procedure. Traditionally, hyperspectral imaging has not been anticipated for intra-operative use in brain surgery because this method has a very limited depth of penetration in tissue and may not be effectively used transcranially.

Further, the narrow corridor in port-based surgery is often occluded when a vessel is accidentally cut. In these incidents, the surgeon may be required to stop his current surgical process (e.g. opening of dura, slight retraction of the sulcus for trans-sulcus navigation of port or resection of tumor tissue) and irrigate the cavity to get a better view of the cavity. Further, such bleeding also limits the surgeon from quickly identifying the location of bleeding so that the particular vessel wall can be coagulated to terminate bleeding.

Accordingly, in some aspects of the present disclosure, systems and methods are provided for utilizing optical imaging in minimally invasive port based surgical procedures. In some embodiments, hyperspectral devices and methods are described for performing intraoperative tissue differentiation and analysis during such procedures.

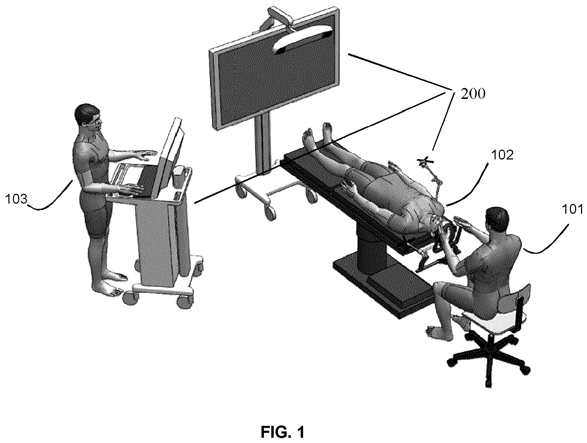

**FIG. 1** shows an example navigation system to support minimally invasive access port-based surgery. **FIG. 1** illustrates a perspective view of a minimally invasive port based surgical procedure. As shown in **FIG. 1**, surgeon **101** conducts a minimally invasive port-based surgery on a patient **102** in an operating room (OR) environment. A navigation system **200** comprising an equipment tower, cameras, displays and tracked instruments assist the surgeon **101** during his procedure. An operator **103** is also present to operate, control and provide assistance for the navigation system **200.**

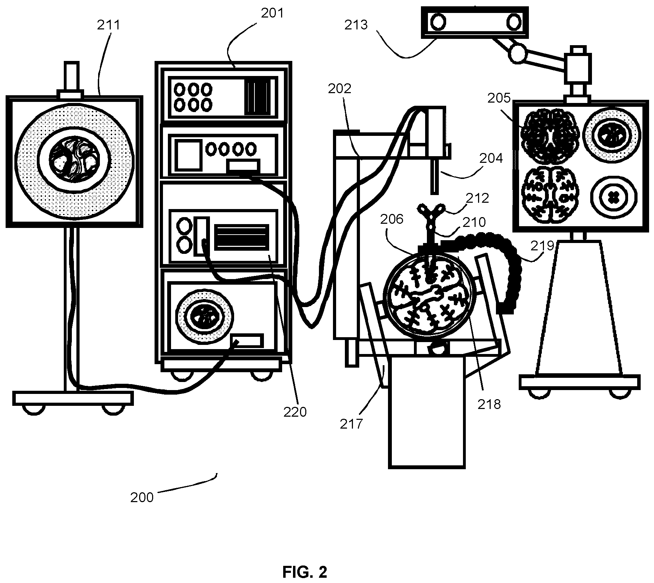

**FIG. 2** is block diagram illustrating system components of an example navigation system. Navigation system **200** in **FIG. 2** includes a monitor **211** for displaying a video image, an equipment tower **201,** a mechanical arm **202,** which supports an optical scope **204.** Equipment tower **201** is mounted on a frame (i.e., a rack or cart) and may contain a computer, planning software, navigation software, a power supply and software to manage the automated arm and tracked instruments. The example embodiment envisions the equipment tower **201** as a single tower configuration with dual displays (**211, 205),** however, other configurations may also exists (i.e., dual tower, single display, etc.). Furthermore, equipment tower **201** may also configured with a UPS (universal power supply) to provide for emergency power, in addition to a regular AC adapter power supply.

Example embodiment **FIG. 2** also envisions equipment tower **201** having recording module **220** that provides real-time recording of the surgical procedure, capturing audio, video, sensory and multi-modal (i.e., CT, MR, US, etc) inputs from different sources. All relevant data is received at equipment tower **201** and stored in memory by recording module **220.** The surgical procedure may be automatically recorded at the outset or be controlled by the operator and / or administrator. In other embodiments, the procedure may be automatically recorded (by default), but there may be an option to override or delete the recording after the procedure has been completed.

The patient's brain is held in place by a head holder **217** and inserted into the head is an access port **206** and introducer **210.** The introducer **210** is tracked using a tracking system **213,** which provides position information for the navigation system **200.** Tracking system **213** may be a 3D optical tracking stereo camera similar to one made by Northern Digital Imaging (NDI). Location data of the mechanical arm **202** and port **206** may be determined by the tracking system **213** by detection of fiducial markers **212** placed on these tools. A secondary display **205** may provide output of the tracking system **213.** The output may be shown in axial, sagittal and coronal views as part of a multi-view display.

Minimally invasive brain surgery using access ports is a recently conceived method of performing surgery on brain tumors previously considered inoperable. In order to introduce an access port into the brain, an introducer **210** with an atraumatic tip may be positioned within the access port and employed to position the access portion within the head. As noted above, the introducer **210** may include fiducial markers **212** for tracking, as presented in **FIG. 2****.** The fiducial markers **212** may be reflective spheres in the case of optical tracking system or pick-up coils in the case of electromagnetic tracking system. The fiducial markers **212** are detected by the tracking system **213** and their respective positions are inferred by the tracking software.

Once inserted into the brain, the introducer **210** may be removed to allow for access to the tissue through the central opening of the access port. However, once introducer **210** is removed, the access port can no longer be tracked. Accordingly, the access port may be indirectly tracked by additional pointing tools configured for identification by the navigation system **200.**

In **FIG. 2****,** a guide clamp **218** for holding the access port **206** may be provided. Guide clamp **218** can optionally engage and disengage with access port 206 without needing to remove the access port from the patient. In some embodiments, the access port can slide up and down within the clamp while in the closed position. A locking mechanism may be attached to or integrated with the guide clamp, and can optionally be actuated with one hand, as described further below.

Referring again to **FIG. 2****,** a small articulated arm **219** may be provided with an attachment point to hold guide clamp **218.** Articulated arm **219** may have up to six degrees of freedom to position guide clamp **218.** Articulated arm **219** may be attached or attachable to a point based on patient head holder **217,** or another suitable patient support, to ensure when locked in place, guide clamp **218** cannot move relative to the patient's head. The interface between guide clamp **218** and articulated arm **219** may be flexible,

or optionally locked into place. Flexibility is desired so the access port can be moved into various positions within the brain, but still rotate about a fixed point.

An example of such a linkage that can achieve this function is a slender bar or rod. When the access port **206** is moved to various positions, the bar or rod will oppose such a bend, and move the access port **206** back to the centered position. Furthermore, an optional collar may be attached to the linkage between the articulated arm, and the access port guide, such that when engaged, the linkage becomes rigid. Currently, no such mechanisms exist to enable positioning an access port in such a manner.

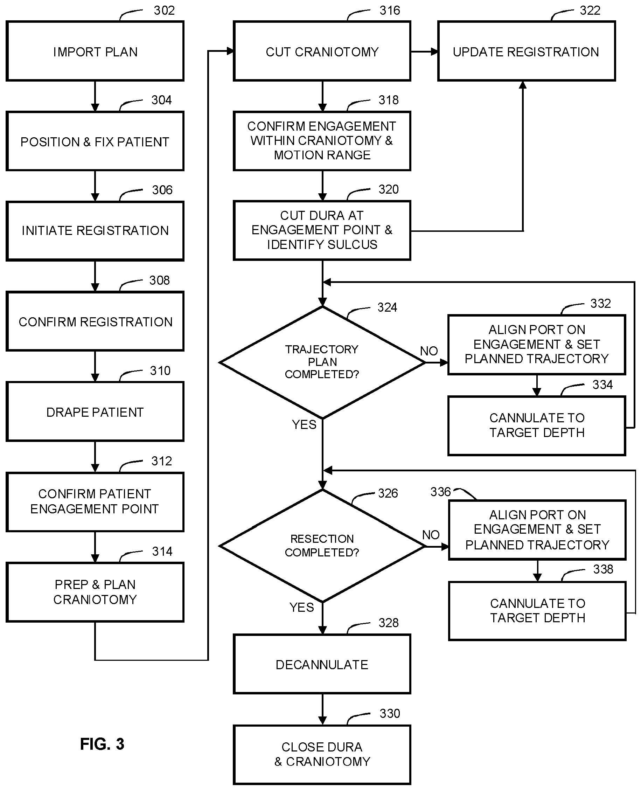

**FIG. 3** is a flow chart illustrating the processing steps involved in a port-based surgical procedure using a navigation system. The first step involves importing the port-based surgical plan (step **302**). A detailed description of the process to create and select a surgical plan is outlined in PCT Patent Application No. PCT/CA2014050272, titled "PLANNING, NAVIGATION AND SIMULATION SYSTEMS AND METHODS FOR MINIMALLY INVASIVE THERAPY".

An example plan, as outlined above, may compose of pre-operative 3D imaging data (i.e., MRI, ultrasound, etc.) and overlaying on it, received inputs (i.e., sulci entry points, target locations, surgical outcome criteria, additional 3D image data information) and displaying one or more trajectory paths based on the calculated score for a projected surgical path. The aforementioned surgical plan may be one example; other surgical plans and / or methods may also be envisioned.

Once the plan has been imported into the navigation system (step **302**), the patient is affixed into position using a head or body holding mechanism. The head position is also confirmed with the patient plan using the navigation software (step **304**).

Returning to **FIG. 3****,** the next step is to initiate registration of the patient (step **306**). The phrase "registration" or "image registration" refers to the process of transforming different sets of data into one coordinate system. Data may be multiple photographs, data from different sensors, times, depths, or viewpoints. The process of "registration" is used in the present application for medical imaging in which images from different imaging modalities are co-registered. Registration is necessary in order to be able to compare or integrate the data obtained from these different modalities.

Those skilled in the art will appreciate that there are numerous registration techniques available and one or more of them may be used in the present application. Non-limiting examples include intensity-based methods which compare intensity patterns in images via correlation metrics, while feature-based methods find correspondence between image features such as points, lines, and contours. Image registration algorithms may also be classified according to the transformation models they use to relate the target image space to the reference image space. Another classification can be made between single-modality and multi-modality methods. Single-modality methods typically register images in the same modality acquired by the same scanner/sensor type, for example, a series of MR images can be co-registered, while multi-modality registration methods are used to register images acquired by different scanner/sensor types, for example in MRI and PET. In the present disclosure multi-modality registration methods are used in medical imaging of the head/brain as images of a subject are frequently obtained from different scanners. Examples include registration of brain CT/MRI images or PET/CT images for tumor localization, registration of contrast-enhanced CT images against non-contrast-enhanced CT images, and registration of ultrasound and CT.

Once registration is confirmed (step **308**), the patient is draped (step **310**). Typically draping involves covering the patient and surrounding areas with a sterile barrier to create and maintain a sterile field during the surgical procedure. The purpose of draping is to eliminate the passage of microorganisms (i.e., bacteria) between non-sterile and sterile areas.

Upon completion of draping (step **310**), the next steps is to confirm patient engagement points (step **312**) and then prep and plan craniotomy (step **314**).

Upon completion of the prep and planning of the craniotomy step (step **312**), the next step is to cut craniotomy (step **314**) where a bone flap is temporarily removed from the skull to access the brain (step **316**). Registration data is updated with the navigation system at this point (step **322**).

The next step is to confirm the engagement within craniotomy and the motion range (step **318**). Once this data is confirmed, the procedure advances to the next step of cutting the dura at the engagement points and identifying the sulcus (step **320**). Registration data is also updated with the navigation system at this point (step **322**).

In an embodiment, by focusing the camera's gaze on the surgical area of interest, this registration update can be manipulated to ensure the best match for that region, while ignoring any non-uniform tissue deformation affecting areas outside of the surgical field (of interest). Additionally, by matching overlay representations of tissue with an actual view of the tissue of interest, the particular tissue representation can be matched to the video image, and thus tending to ensure registration of the tissue of interest.

For example, video of post craniotomy brain (i.e. brain exposed) can be matched with an imaged sulcal map; the video position of exposed vessels can be matched with image segmentation of vessels; the video position of a lesion or tumor can be matched with image segmentation of tumor; and/or a video image from endoscopy within a nasal cavity can be matched with bone rendering of bone surface on nasal cavity for endonasal alignment.

In other embodiments, multiple cameras can be used and overlaid with tracked instrument(s) views, and thus allowing multiple views of the data and overlays to be presented at the same time, which can tend to provide even greater confidence in a registration, or correction in more than dimensions / views.

Thereafter, the cannulation process is initiated (step **324**). Cannulation involves inserting a port into the brain, typically along a sulci path as identified in step **320,** along a trajectory plan. Cannulation is an iterative process that involves repeating the steps of aligning the port on engagement and setting the planned trajectory (step **332**) and then cannulating to the target depth (step **334**) until the complete trajectory plan is executed (step **324**).

Returning to **FIG. 3****,** the surgeon then performs resection (step **326**) to remove part of the brain and / or tumor of interest. Resection (step **326**) is a continual loop including both fine and gross resection (step **336**). The next step involves hyperspectral imaging (step **338**) which may be performed on either fine or gross resection (step **336**). Hyperspectral imaging (step **338**) is used as a form of tissue differentiation and may assist surgeons to investigate cancerous stem cells. Further, the ability to hyperspectrally image tissue being operated on either as part of an external video scope or as a separate module may provide the ability to perform chemical imaging using the absorption of tissue, the ability to differentiate tissues based on scattering properties, and / or the ability to improve visualization by imaging at wavelengths with reduced absorption or scattering properties.

Once resection is completed (step **326**), the surgeon then decannulates (step **328**) by removing the port and any tracking instruments from the brain. Finally, the surgeon closes the dura and completes the craniotomy (step **330**).

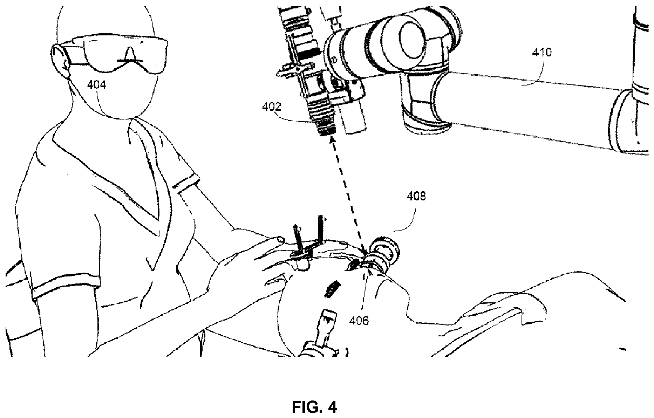

**FIG. 4** illustrates an example port-based brain surgery procedure using a video scope. In **FIG. 4****,** operator **404,** typically a surgeon, would align video scope **402** to peer down port **406.** Video scope **402** may be attached to an adjustable mechanical arm **410.** Port **406** may have a tracking tool **408** attached to it where tracking tool **408** is tracked by a tracking system of a navigation system.

Even though the video scope **402** is commonly an endoscope or a microscope, these devices introduce optical and ergonomic limitations when the surgical procedure is conducted over a confined space and conducted over a prolonged period such as the case with minimally invasive brain surgery.

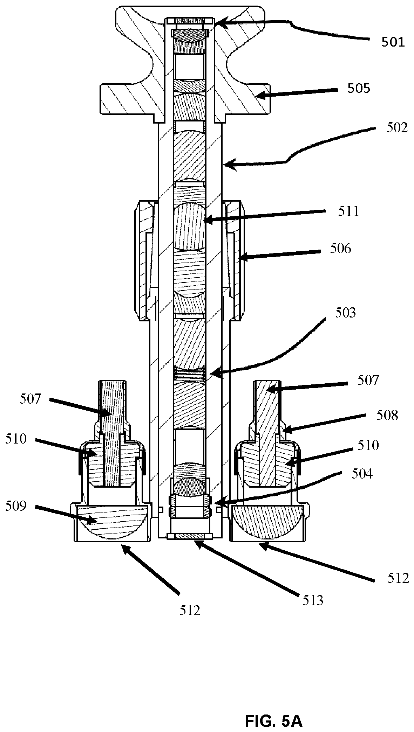

**FIG. 5A** illustrates the design of a video scope that is composed of a lens assembly **511** (explained later) and two illumination delivery points 512. The lens assembly **511** is terminated at the eyepiece end with a sealed window **501** at the proximal end. Sealed window **501** is typically made of quartz, to help maintain water seal since OR devices must be steam cleanable. The eyepiece end also has a camera coupler **505** that provides a standardized mounting point for a camera (not shown). The camera may be a standard definition (SD), high definition (HD) or ultra high definition (UHD) camera. In another embodiment, the camera may be replaced by other imaging technologies such as Optical Coherence Tomography (OCT) or Polarization Sensitive-OCT. The distal end of the lens assembly is also sealed with a clear window **513** at the distal end. The distal end also supports illumination optics **512.** In an alternate embodiment the distal end may be also optionally affixed with a polarizing filter to enable polarization sensitive imaging.

The PS OCT technique described herein may be used to specifically visualize tissue exhibiting structural organization. Examples of such tissue structures include tendons that are attached to bones. Other examples of tissue that exhibit structural organization include ligaments, muscle, cartilage, tissue connective membrane, nerves, retina, blood vessel walls, some bone structures, trachea, esophagus, tongue and teeth. PS OCT commonly generates a heat map or pseudo colored image (reference: "Correlation of collagen organization with polarization sensitive imaging of in vitro cartilage: implications for osteoarthritis," W. Drexler et.al, The Journal of Rheumatology, Vol. 28, No. 6, 1311-1318) where tissue structures with high degree of organization appear highlighted. Hence the system can be used in orthopedic surgery to visualize tendons and optionally avoid unintentional damage to this tissue during a procedure. These identified regions of tissue exhibiting high level of structural organization (e.g. tendons and ligaments that are often located near skeletal structure) may be used in conjunction with a priori information, such as known points of attachment of tendons to bones, to geometrically correlate PS OCT images to CT and MR images where bones are easily imaged.

The insertion sites, tendon-bone junctions and ligament-bone junctions, are known as entheses. The anatomical locations of entheses are well known and landmarks can be identified on the bone in the vicinity of these attachment points (reference: "Anatomy and biochemistry of enthuses," Michael Benjamin, Ann Rheum Dis 2000, Vol. 59, Issue 12, pg:995-999). Hence, this *a priori* anatomical information about the position of the tendon or ligament relative to bone structures in the vicinity can be used to register intraopertive PS-OCT image of the tendons or ligaments with pre-operative images obtained using other modalities that accurately image the bone structures.

For example the tendon-bone junction in the Achilles tendon enthesis is immediately proximal to the superior tuberosity. This region is characterized by a highly irregular interface at the attachment points or junction. This characteristic structure of the bone can be used to identify the junction where the tendon attaches to the bone. The geometric correlation of images that are thus obtained using different modalities, and often at different scales, is known as image registration or image fusion.

Common methods for multi-modal image registration mentioned above include those described in "Multi-modal image registration for pre-operative planning and image guided neurosurgical procedures," Risholm, et.al, Neurosurg Clin N Am, 2011, April; 22(2): 197-206 and "Image registration of ex-vivo MRI to sparsely sectioned histology of hippocampal and neocortical temporal lobe speciments," Goubran et.al, Neurolmage, 83 (2013); 770-781. Broad classes of image registration methods for medical images is also described in detail in "A survey of medical image registration," Maintz et.al, Medical Image Analysis (1998), Vol. 2, No. 1, pp: 1-36.

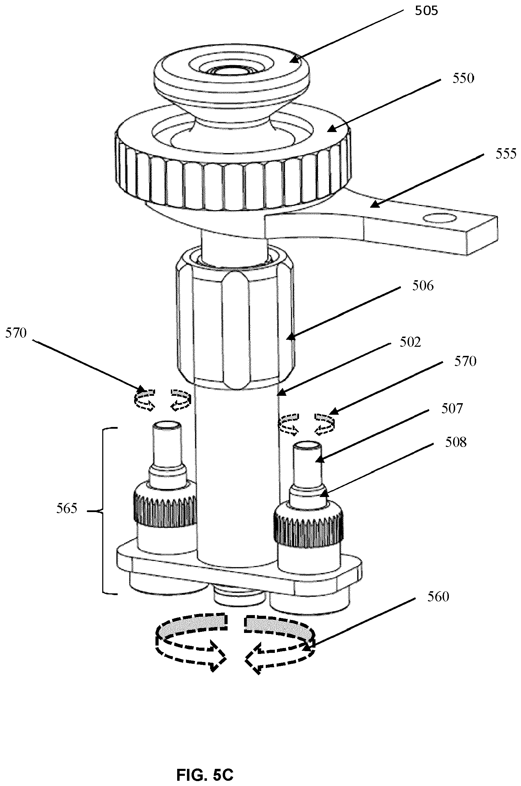

The illumination optics is comprised of fiber bundles **507** that are rotatably attached using a pair of connectors **510.** The connectors **510** allow the fiber bundles to rotate freely (**570** in **FIG. 5C**) within the connector while maintaining a fixed distance between the lens **509** and tip of the fiber bundle **507** using a loose sleeve **508.** This rotation movement will reduce the strain on the fiber bundle when the video scope is moved on a holding system (not shown) or a mechanical arm **410** as seen in **FIG. 4****.** The rotatable connector **510** also aid in easy cable management when the mechanical arm **410** is moved during a surgical procedure. The illumination optics are placed as close as possible to the objective lens. One non-limiting example of spacing between the optics is approximately 30 to 35 mm, or 32 to 34 mm, between the center of the lenses **509** where the diameter of lenses **509** is approximately 15mm. This configuration is optimal for illuminating the bottom of a surgical port with maximum intensity when the distal end of the video scope is between 25cm to 40cm from the bottom of the surgical port. An optical compensator **503** is used to act as a thermal compensator to control the stress on optical components during steam cleaning. A holder **506** provides an easy to grasp assembly to hold and manipulate the video scope without introducing mechanical stress on the lens assembly. The lens assembly is encased in a sealed barrel **511** to avoid ingression of steam and liquids during normal use and cleaning. The rotatable attachment mechanism **510** allows free rotation of the fiber bundles when the camera is moved manually or when mounted to a robotic positioning system. This, in turn, avoids undue stress on the fiber bundles that are susceptible to fracture.

**FIG. 5C** illustrates a non-limiting example to realize a functionality that allows the illumination assembly **565** to rotate radially **560** around the video scope barrel **502.** The illumination assembly **565** is composed of the two fiber bundles **507** on either side of the video scope, mounting mechanism **508** and lens **509** (as in **FIG. 5A**). This allows the surgeon to adjust the radial orientation of the illumination and orient the illumination assembly so that it minimally obstructs the surgeon's view of the surgical space. The illumination assembly can be freely rotated without rotating the video scope by securing the video scope to an external positioning mechanism, such as **410,** using a removable clamp **555** and an associated **lock 550.** The removable clamp's distal end **555** and the associated lock **550** may be mated together using a thread mechanism or any other mechanical fastening mechanism. The removal clamp's proximal end (not shown) may be secured to the external positioning mechanism **410.** It should be further noted that the rotation **560** enabled in this design along with the rotation **570** of the fiber bundles within the connectors enable positioning and orientation of the video scope with minimal interruption of the visible surgical space and minimize strain on the fiber bundles during motion. Finally, the illumination assembly 565 may be replaced with alternative configurations such as ring lights or single illumination points. Ring lights may be realized through circular arrangement of fiber strands (not shown) from an optical fiber bundle around the circumference of the objective lens. Single illumination points may be realized through removal of one of the two split fiber bundles **507** from the design.

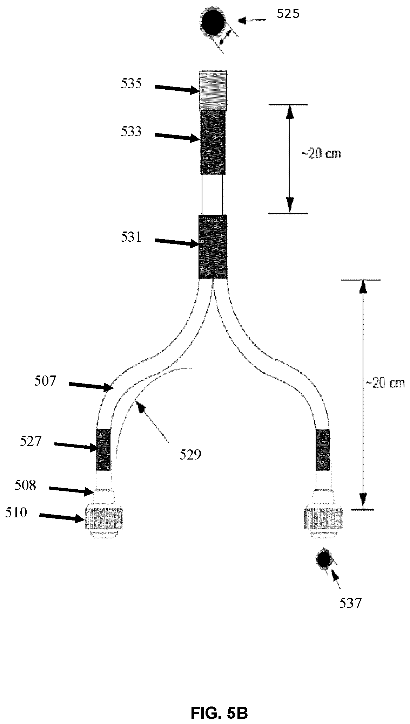

The illumination assembly preferably receives the light input from an optical source that is located away from the video scope. This reduces the total weight of the external scope and allows for easy manipulation of the video scope by a manual positioning system (not shown) or a mechanical arm 410. The light from the light source is delivered to the video scope through the use of a fiber bundle. Presence of two delivery points represented by illumination optics **512** in **FIG. 5A** requires the use of a fiber bundle that is split in two. This design of fiber bundle is also known as a Y-cables. An example embodiment of this Y-cable design is illustrated in **FIG. 5B****.** In **FIG. 5B****,** rotatable connections **508** are provided on the fasteners **510** at the two distal end of the Y cable, providing a mechanism for freely rotating the fiber bundles to avoid fracture of the bundles. A strain-relief **527** helps maintain a minimum limit on the bend radius **529** of the bundle between the two distal ends and the Y-junction **531.** Y-junction **531** helps reduce bend strain on the fiber bundle **507.** Strain-relief **533** similarly aids in reducing bend strain near the connector **535** at the proximal end of the Y-cable. Cross sections **525** and **537** illustrate fiber bundles at the two ends of the Y-cable. The length of the cable may be at least 40 cm with the Y-junction **531** placed equidistant from the two ends. This dimension provides for placement of light source on a cart or instrumentation tower **201** sufficiently away from the mechanical arm **410** while minimizing light loss due to excessive length of the fiber bundle.

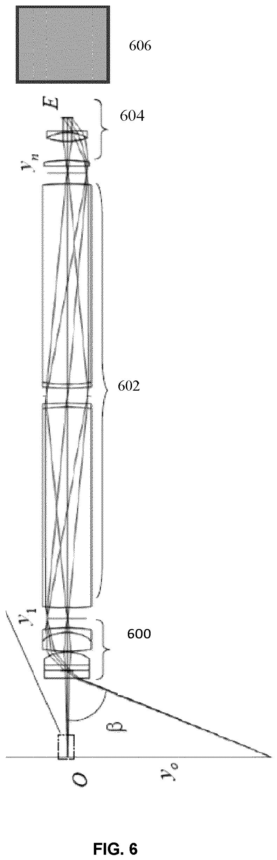

**FIG.** 6 illustrates an optical design of the video scope that limits the diameter of the objective lens **600** (front lens). This design enables the mounting of illumination optics immediately adjacent to the objective lens so that the illumination beam can be almost collinear to the return path of the light reflected from the tissue. The illumination beam and the reflected beam need to be as collinear as possible so that maximum illumination is delivered at the bottom of the access port **406.** Finally, the optical design is constrained so that the length of the lens assembly is minimized to make the whole video scope **402** minimally intrusive to the surgeon's field of view and facilitate easy access to the surgical space by the surgeon. This constraint is a challenge in conventional optical design conventional optical design techniques maximize zoom by utilizing maximum available physical length of the lens assembly during the design process. This optical design of the present disclosure is adapted from a conventional endoscopic system that consists of objective lens **600,** relay lens **602** and eyepiece **604.** The zoom parameter of the optical assembly is chosen such that the minimum field of view (corresponding to maximum zoom) is equal to approximately 13mm. This dimension is the diameter of the surgical port. The field of view of 13mm needs to be achieved at a minimum working distance of 25 cm where the minimum working distance is defined as the distance between the distal end of the video scope (**402** in **FIG. 4**) and bottom of the surgical port (**406** in **FIG. 4**). As explained in **FIG. 5A****,** a coupler **505** is used to attach a camera at the eyepiece end (marked 'E' in **FIG. 6**). The optical design of the objective is composed of 1 doublet and 1 singlet; the relay is composed of 1 doublet and 1 singlet and the eyepiece is composed of 2 singlet and 1 doublet. Any manufacturing error is compensated using one optical compensator **503** that is placed between the objective and relay. The length of the optical sub-assembly is minimized through the use of higher power lenses and fewer lens groups.

The type of surgical procedure determines either a wide-field of view (WFOV) or a narrow field of view (NFOV) video scope. For example, a neck surgery may benefit from a WFOV video scope where large area is captured by the video scope; whereas, a port-based brain surgery may benefit from a NFOV video scope. Instead of attempting to address both these design requirements using one device, two separate designs may be developed such that they share several sub-components and the manufacturing process. Hence, it is economical to manufacture two different designs while sharing number of design elements and assembly procedure. Both WFOV and NFOV designs share a similar optical illumination system 512 as seen in **FIG. 5A****,** The WFOV design can be realized by attaching a camera to the camera coupler **505.** The zoom adjustment of the camera is used to determine the field of view in this case.

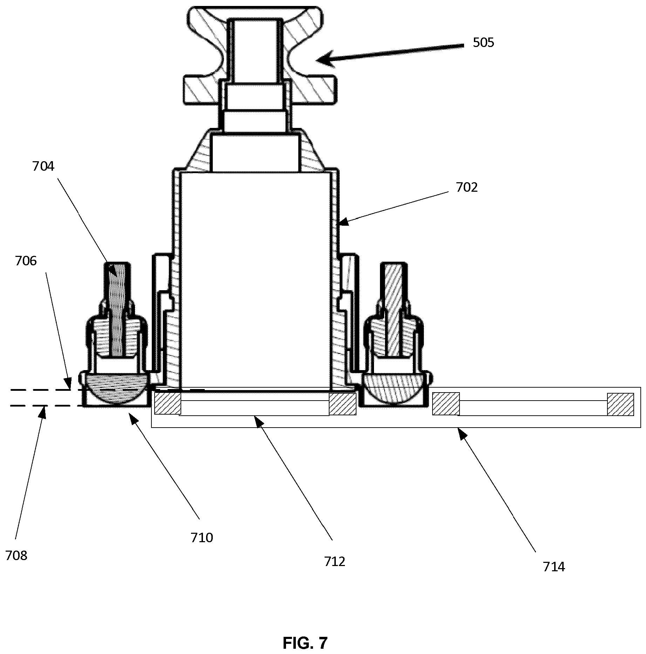

**FIG. 7** illustrates an assembly with a non-coaxial illumination source. The illumination system **710** is similar in design to that illustrated in **FIG. 5A** and consists of fiber bundles **704** (only a distal portion of which are shown in the Figure). An air-filed opaque tube (also known as optical tube) **702** is used to position the illumination mechanism away from the camera attached to the coupler **505.** It should be noted that any required magnification may be provided by the camera lens (not shown but typically attached to the camera coupler) for WFOV application. A finite space that is at least 1 mm between the plane **706** of the distal end of the optical tube and the plane of the illumination optics **708** helps isolate the illumination light from directly reaching the camera input. It should be further noted that the dimensions of the WFOV optics will be such that the illumination will not be nearly coaxial with the path of the reflected light. This is not a limitation in this configuration because WFOV is used to observe a surgical space that is larger that of a port (which is approximately 13mm). Hence, general illumination is sufficient. Placement of the illumination source close to the camera does improve illumination of the surgical area compared to the use of overhead surgical lights and avoids glare from area outside of the surgical space. The role of additional components, **712** and **714,** are explained below in the context of hyperspectral imaging.

In another embodiment of the video scope, the illumination sources placed immediately adjacent to the distal end of the video scope may be employ a light source such as luminance light emitting diodes or Super Luminescent Diodes (SLD's) (not shown). Since the light sources are not coaxial to the reflected light path (the light path incident on the lens and camera assembly), the light sources have to be aimed or steered at the focal plane of interest. Such steering may be achieved using movable fiber bundle mounts **510** as shown in **FIG. 5A****.**

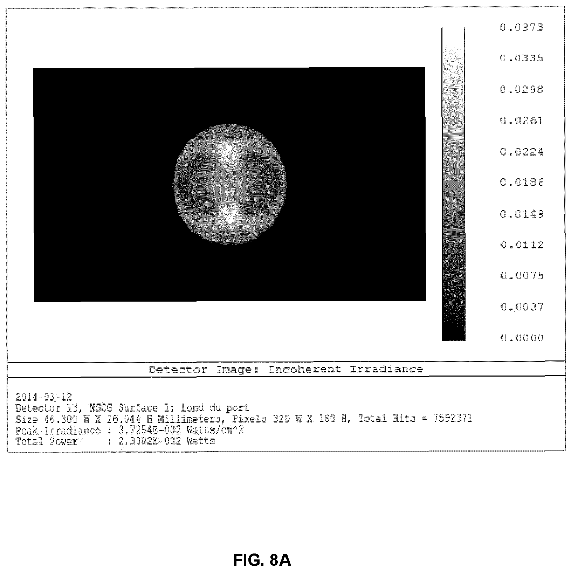

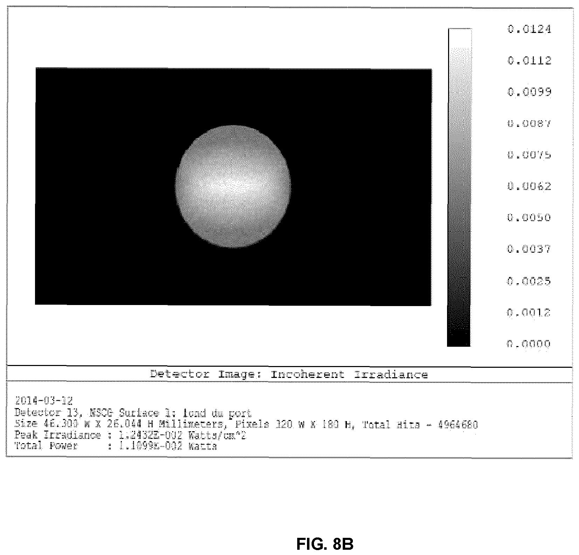

Application of such externally positioned illumination sources in port-based imaging introduces several challenges. First, the walls of the port are either partially or fully reflective. This introduces localized regions in the imaged surface that have higher intensity of incident light. Such regions are commonly known as hot-spots. It is desirable to avoid such high intensity regions as these tend to saturate sensors and, hence, limit the dynamic range of the sensors in the camera mechanism. Use of post-processing to normalize intensities is less optimal as saturation of sensors results in information loss that cannot be recovered. Presence of high intensity regions can be reduced through the use of surface textures on the port walls that diffuse the light. The impact of using smooth and rough surface texture on the port walls is illustrated in **FIGS. 8A** and **8B****,** respectively. The reflections resulting from textured walls is referred to as Lambertian reflection. The assessment presented in **FIGS. 8A** and **8B** were conducted using ray-tracing tools and the resulting intensity of light at the surface of the tissue (distal end of the port) were visualized using heat-maps or pseudo color where high intensity corresponded to white and low intensity corresponded to black.

Another approach to uniformly illuminating at the bottom of the port is to model the light rays using a commonly known optical modelling method, such as ray tracing, and establish the optimal orientation of the light sources that minimize hot-spots at the bottom of the surgical port. Orientation of the light sources may be modified using a beam steering mechanism, such as the one illustrated in **FIG. 5A****.** Alternatively, a robotic positioning system may be used to achieve this steering.

Port-based imaging is also limited by highly reflective nature of some but not all regions of the brain tissue due to the presence of blood, CSF or other fluids. In the latter case, an initial image could be acquired to identify regions with high intensity reflected light and this information can be used to reposition direction of the light sources in an attempt uniformly distribute the incident light. As described above, imaging using white light has several challenges in the operating room. Several of these challenges can be overcome by limiting the spectral range of the light that is observed or by judiciously combining selected wavelength bands to visualize human tissue in the operating room.

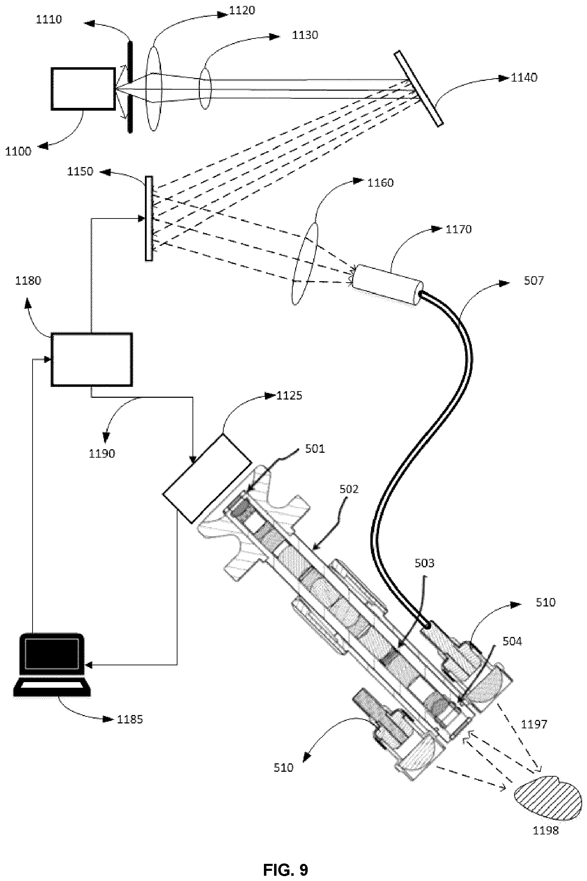

**FIG. 9** illustrates a video scope that has been adapted to accommodate hyperspectral imaging capabilities. In this embodiment, tunable light source that is adapted based on the surgical context e.g. selection of illumination spectral region where blood is highly absorptive (to detect blood clots) or transmissive (to avoid excessive light scattering) may be used.

**FIG. 9** illustrates one such system. The tunable light source is mainly composed of a broadband light source **1100,** a spectral separation mechanism **1140,** a spectral filtering mechanism **1150** and a mechanism to combine the filtered frequency bands **1170.** The combining mechanism consists of a lens and a fiber bundle that mixes all the reflected wavelength bands into one beam that is transmitted through the fiber bundle **507.** The light from light source **1100** is passed through a slit **1110** to generate a narrow beam. This light is then collimated using optical elements **1120** and **1130.** The collimated beam is then split into its spectral components using a prism (not shown), reflective or transmission grating.

**FIG. 9** illustrates the use of a reflective grating **1140.** The spatially separated beam is filtered by selectively reflecting portions of the spatially separated beam. This is achieved using a spatial light modulator, SLM **1150,** such as a Digital Light Processor (Texas Instruments Inc). An SLM is composed of an array of micro-mirrors that can be electronically activated to act as mirrors or deactivated to acts as opaque surfaces. Hence, specific portions of the spectrum are reflected while other regions are suppressed based on the pattern of activated micro-mirrors. The beam that is now composed of selective portions of spectrum are combined using focusing optics **1160** and a combiner **1170.**

The recombined beam is now composed of only those wavelengths that were selectively reflected by the spatial light modulator, SLM **1150.** This light can be used as the illumination source of an imaging system or external scope by transmitting the light via a light pipe **507** to the illuminator connector and lens mechanism **510** attached to the external scope. It should be noted that the video scope illustrated in **FIG. 9** shows the connection of light pipe **507** to only one of the two illuminator connectors **510** for the sake of simplicity of the illustration. Details of connecting the light pipe to the video scope is further explained in **FIG. 5A****.**

The reflected light from the tissue **1198** is captured by the external scope that is composed of lens assembly **502.** As detailed in **FIG. 5A****,** the lens assembly is composed; this light is captured using a high resolution detector **1125** that is usually a charge coupled device, CCD. The specific band of wavelengths that are reflected by the SLM are controlled by an SLM controller **1180** that is under the command of a computer **1185.** The same computer is used to acquire the image from the detector **1125.** Hence, the computer can synchronize the illumination of a material **1198** with a specific wavelength band or wavelength bands of light and acquire corresponding reflected light. This association of illumination wavelength and acquired image can be used to construct a hyper-spectral image where each image is a 2D or 1D image and the third dimension is an index that corresponds to illumination wavelength band(s). Since the individual micro-mirrors located in an SLM can be operated at a rate as high as 4kHz, subsequent frames of the field of view can be obtained at different wavelength bands.

Further, some of the acquired frames can be for employed white-light illumination of the tissue. This is possible by operating the acquisition camera at a frame rate that is sufficiently high to provide smooth video playback, as perceived by a human observer when white light frames are intermittently obtained while collecting hyperspectral image frames. For example, in some non-limiting examples, the frame rate may be selected to be higher than 20 frames per second, higher than 24 frames per second, or higher than 30 frames per second, in order to support white light video acquisition at such frame rates while obtaining hyperspectral data. For example, at a camera frame rate higher than 20 fps, a white-light image can be acquired every 1/20^{th} of a second and any additional frame can be allocated for acquisition using specific wavelength bands. A white light video feed may then be separately generated and displayed based on the collected white light images. This allows the surgeon to continuous view a white-light image of the surgical area while acquiring any additional images at different wavelength bands in a multiplexed manner. The white-light image stream (or video) may be viewed in one display or sub-section of a display and other images acquired using other wavelength bands may be viewed in a second display or second sub-section of the same display.

The individual wavelength bands can be composed of non-overlapping individual wavelength bands or combination of bands that may overlap. Alternatively, at least one of the acquired frame can correspond to illumination **1197** of the subject material **1198** using the entire wavelength band of the light source. The entire wavelength band could be also normalized to ensure that all the intensity in the output light emanating from the combiner **1170** is consistent across the entire spectrum. This is known as white balancing. In summary, the same optical mechanism can be used to acquire hyperspectral images and white-light images that are interspersed among each other in the acquired sequence of images. This embodiment eliminates the need for splitting the acquired beam into separate paths so that one beam is captured by a hyperspectral imaging system while the other beam is captured by a white-light camera. This reduces the design complexity of the optical system and aids in making the system more compact as the spectral shaping part of the system can be separated from the imaging system using a light pipe to channel the output light from the light source. It should be noted that the sample being imaged **1198** may be an ex-vivo tissue sample or portion of the brain tissue that may be exposed through a port-based neurosurgical access inserted in the skull.

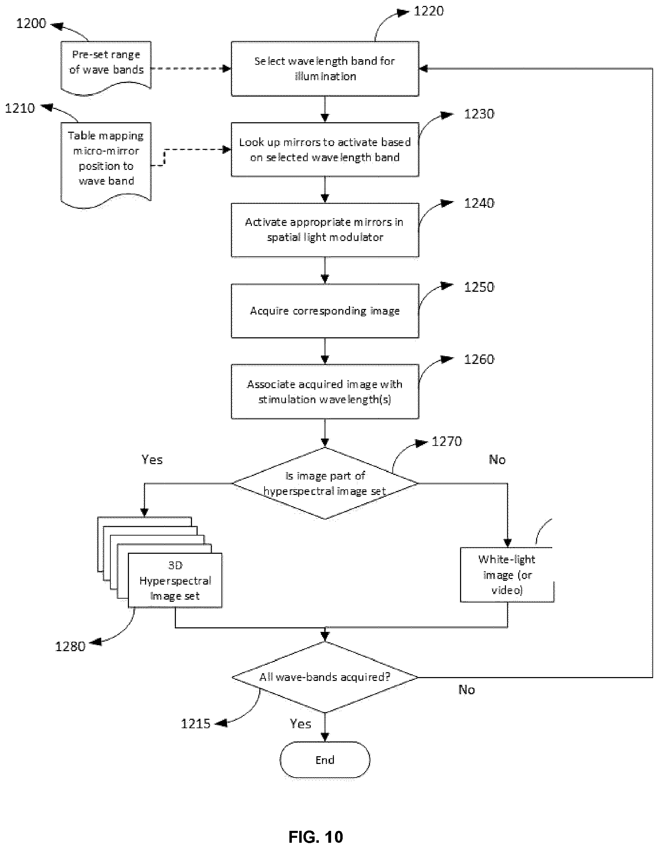

The software system used to acquire hyperspectral data and white-light images (or video) in a multiplex fashion is illustrated in **FIG. 10****.** First the range of wavelengths (wave bands) that are of interest are stored in a table (step **1200**). Then, specific wave band for illumination is selected from the table (step **1220**). Each entry in this table is used to look up (step **1230**) specific micro-mirrors that need to be activated using another table (step **1210**). Hence, only the micro-mirrors associated with specific wavelength bands are activated (step **1240**). Activation of a micro-mirror turns it into a micro-reflector instead of an opaque surface. Hence, the sample **1198** in **FIG. 9**) is illuminated with light (**1197** in **FIG. 9**) that is composed of specific wavelength bands. The table (step **1200**) may also include entries that activate the entire spatial light modulator (SLM). In this case, the SLM acts as a mirror for the entire bandwidth of the light source and the acquired image will correspond to white-light illumination.

Returning to **FIG. 10****,** the reflected light from the illuminated sample is acquired (step **1250**) by the same computer and associated with the specific wavelength band (step **1260**). The type of illumination (white-light versus specific wavelength band) used for each acquired image is interrogated (step **1270)** in order to appropriately classify the acquired image as part of white-light image (video) or part of the hyperspectral image data set. If the acquired image corresponds to a narrow wavelength band then it is stored as part of the hyperspectral image set (step **1280**). If the image corresponds to white-light illumination, it is stored as white-light image or a stream of such images may be captured to represent a video stream. This acquisition is repeated (step **1215**) until all the wavelength bands of interested are sequentially used to illuminate the sample material. Hence, the resulting image set will be composed of both hyperspectral image sets (step **1280**) and white-light image sets (step **1290**), all acquired using the same hardware.

Ideally, the video stream needs to be at least 30 frames per second to provide a flicker-free video to the surgeon. If a total of 40 frames are acquired per second, the additional 10 frames may be used to store images corresponding to 10 distinct or overlapping wavelength bands. Hence, if the total frame rate of the acquisition system is n frames per second, n-30 frames may be allocated towards n-30 wavelength bands in the hyperspectral image data set.

An alternative to tunable light source 1110 shown in **FIG. 9** may be monochromatic, spanning ultra violet (UV), visible, and/or near infrared (NIR) wavelengths, continuous wave or pulsed that is used to illuminate the tissue using free space or fiber coupled mechanism

In another embodiment, specific wavelength bands may be acquired by filtering the reflected light from a broadband light source using such spectral elements as discrete wavelength filters (on filter wheel or spatial on-chip filters), liquid crystal filters, spectrographs/spectrometers/spectral gratings, spatially varying gratings, fiber-coupled spectrometers.

**FIG. 7** also illustrates the implementation of discrete filters **712** attached to a rotatable filter wheel **714**) that may be motorized. This filter mechanism is attached at the distal end of the video scope. Another alternative to discrete filters at the input to the video scope is a liquid crystal-based tunable wavelength filter (not shown) to pass only a narrow range of wavelengths. This filter can be tuned to a number of different wavelengths and operates in a similar manner to the discrete filters as an image is acquired for each wavelength the filter is tuned to. In yet another embodiment, diffraction grating based systems that separate input light input its constituent wavelengths may be used in lieu of the camera **1125** shown in **FIG. 9****.** Imaging spectrometer systems rely on scanning the entrance slit of the system across the field to be imaged. Thus the acquisition time is limited by the scanning time. The entrance slit of the spectrometer can be either free space or fiber coupled to the optical path. If an array-to-line fiber mapping is utilized it is possible to acquire all spatial and spectral information simultaneously. The spectrometer could be alternatively equipped with Spatially Varying Gratings where a specialized diffraction grating that allows for the collection of spectra from all pixels in a single acquisition. The grating is divided into a number of spatial gratings each with a varying direction of diffraction. An image is acquired that captures the diffracted light from each of these grating regions, this image is then reconstructed to form the hyperspectral data set.

Non-limiting examples of camera **1125** include monochrome video camera with resolution up to high definition (HD) or ultra high definition (UHD). CCD, CMOS, InGaAs, or HgCdTe device.

Another aspect of confocal hyperspectral imaging system is that the entire tissue surface does not have to be scanned in a raster pattern. Instead, random spots can be accumulated until a reasonable match is found against pre-defined data classes. This can significantly reduce the data acquisition time associated with hyperspectral imaging.

In some embodiments, the hyperspectral imaging system illuminates the tissue with monochromatic or broadband light, collects light reflected from the tissue, controls the wavelength of the detected light in such a way that a series of images, each recorded at different wavelengths or wavelength ranges, is collected. This series of images, known as a hyperspectral dataset, is processed to extract tissue's bio-chemical or microstructural metrics and reduced to 2D (spatial). This reduced 2D image may be spatially registered
and can be overlaid on the external video scope image as well as any other pre- and intra-operative images. For example, methods of correlating image data are disclosed in PCT Patent Application No. PCT/CA2014/050269, titled **"INTRAMODAL SYNCHRONIZATION OF SURGICAL DATA"** and filed on March 14^{th}, 2014. Spatial registration is realized by using navigation markers attached directly on the camera or on structures rigidly and consistently attached to the camera. This provides both location and orientation of the imaging system. This is further explained in the disclosure related to automated guidance of imaging system.

The hyperspectral dataset **1280** in **FIG. 10** is then processed to extract the tissue specific information and reduce the dimensionality of the data. Tissue specific information can range from tissue type identification to inferring pathology associated with a region of the acquired image. Examples of the possible processing methods including the following:
In one embodiment, if the spectral peaks or features of chemical(s) of interest are known, the spectra and be processed, through either peak or feature detection algorithms, to detected the peaks or features to give an indication of the chemical presence and some indication of the concentration or quality. This useful only if the specific chemicals of interest are known.
In one embodiment, the spectra of specific tissues or tissue states of interest can be acquired and stored in a database, as disclosed in PCT Patent Application No. PCT/CA2014/050269, titled "INTRAMODAL SYNCHRONIZATION OF SURGICAL DATA" and filed on March 14^{th}, 2014. Spectra then acquired during the surgery can be compared to the spectra stored in the database for similarity and if sufficiently similar to give an indication of what tissue or tissue type the spectra was acquired from.

Multivariate/chemometric methods, which are a wide grouping of statistical techniques where a method is trained on spectra collected from samples with known states (i.e., spectrum and corresponding chemical level, tissue type, tissue state, etc.), may be used to predict the state of a new sample based on the acquired spectrum. Some of the more commonly used employed techniques include principal component regression (PCR), partial least squares (PLS), and neural networks (NN).

The aforementioned analysis methods can be implemented in a computer system, and hence the results of the analysis can be obtained in near-real time for appropriate use by a surgeon. This may significantly reduce the need for similar analysis by a pathologist and reduces the wait time associated with obtaining results of such tissue analysis. Correlation metrics between newly acquired data and representative data in a knowledge-base (or database or training set) provide the surgeons a means of quantifying tissue types. Such metrics may be a representation of confidence associated with automated inference provided by the software algorithm.

Finally, the ability to selectively view narrow bands of the spectra or reject narrow bands of the spectra may allow the surgeon to reject bright reflections from blood. Hence, the surgeon may be able to view the interior of the corridor and proceed with surgical resection of tumor even when the corridor is occluded by excessive bleeding. This will reduce the need to constantly irrigate the narrow corridor and hence reduce interruption of the surgical procedure.

In another embodiment the optical characteristics and chemical composition of blood may be taken advantage of to visualize vasculature located at or immediately below tissue surface. For example, a common challenge associated with the dural opening step in cranial surgery is the inability to anticipate the presence of vasculature immediately below the dura. Hyperspectral imaging (HSI), with emphasis on red and near infrared portions of the imaging spectrum, can be used to selectively visualize regions with high haemoglobin content. Due to the blood-brain barrier that naturally exists in the human brain, this technique will result in selective imaging of vasculature.

An example method for visualizing vascular structures located below other tissue structures is disclosed in "Characterization of vascular structures and skin bruises using hyperspectral imaging, image analysis and diffusion theory," L.L. Randeberg et.al., Journal of Biophotonics, No. 1-2, 53-65 (2010). Another application of hyperspectral imaging, such as that enabled by invention presented in this document, is the reliance on reflectivity of hemoglobin that is particularly strong in tumor microvasculature. This is described in detailed in "Hyperspectral imaging of hemoglobin saturation in tumor microvasculature and tumor hypoxia development," B.S Sorg, et.al., Journal of Biomedical Optics, 10(4), July/August 2005.

Similarly, OCT based imaging may be also used to visualize structures located immediately below the dura since the dura is not more than a millimetre thick. Vasculature is one of the predominant structures located below the dura. Others structures of relevance that can be similarly imaged include the sulcal folds.

The vasculature that is visualized using the techniques described in the preceding paragraphs is a map of the structure of the blood vessels in the vicinity of the region being imaged. Such regions may include the region in the vicinity of the trajectory along which a port is inserted in port-based cranial surgery. This vascular structure in the surgical region will be referred to as the in-situ vascular structure or intra-operative local vasculature. This in-situ geometry of the vascular structure may be then be compared with whole-head vascular structure images derived from MRI acquired after the injection of Gadolinium or other contrast medium in the veins. The latter whole-head images are obtained prior to a surgical procedure and referred to as pre-operative vascular structures. The pre-operatively vascular structures are imaged relative to the anatomy of the patient. In other words, the location and orientation of this vascular structure is known relative to the anatomy of the patient.

This comparison of in-situ (intraoperative) vascular structure with pre-operative vascular structures may be used to infer the exact location of the in-situ vascular structures relative to the pre-operative vascular structures. This allows the surgeon to confirm or infer their current surgical position relative to the anatomy of the patient. Hence, the vascular structure can be used as a land-mark or reference frame for navigated surgical procedures. Although the above illustration is in the context of cranial surgery, it will be appreciated that the method can be extended to any other part of the anatomy where vasculature can be visualized pre-operatively and intra-operatively. Example procedures include, retinal surgery and lung biopsy to mention just a few.

Geometric correlation of the local vascular structure with the global vascular structure is feasible because the anatomy and pattern of blood vessels is known (reference: "Cortical blood vessels of the human brain," Duvernoy et.al., Brain Research Bulletin, Vol. 7, Issue 5, November 1981, pg 519-579 and "A computed tomographic guide to the identification of cerebral vascular territories," Damasio et.al., Arch Neurol, 1983; 40(3): 138-142. Thus while it is known that registration, in general, involves the geometric correlation of small scale image (or 3D structure) with a large scale image (or 3D structure); however, this geometric correlation involves the use of features that are common between the two image sets. The use of the vasculature by itself as a feature is as disclosed herein is the first instance of this. The vasculature can be used as a feature because of its unique structure and this uniqueness is well known (reference: see two papers cited in this paragraph). Unique vascular structure is known to exist in several regions of the body, for example the cerebral region, retina and the cardiac regions.

Thus, using the intraoperative hyperspectral image data, a local vascular structure in the selected local region of the tissue is identified, and then the global vascular image data is searched for identifying and locating a portion of the global vascular structure geometrically matching the local vascular structure, and upon identifying and locating matching vascular structure, geometrically correlating and registering the local vascular structure in the selected local region of the tissue with the a global vascular structure within the tissue for confirming location of the local vasculature structures. The location, thus inferred, is invaluable for guiding tools in the local region for surgical procedures. Such surgical procedure is known as navigated surgery.

It is noted that embodiments provided herein may employ software to process the 3D dimensional data sets to extract the information of interest, and to reduce the data to a 2D image that can be visualized in conjunction with or overlaid on the surgical image acquired by the external video scope. These software methods could include everything from simple spectral peak detection to more sophisticated multivariate, chemometric, and data mining techniques to extract the metric of interest from the acquire spectra. The spectrum associated with each pixel may be processed according to such methods.

As hyperspectral imaging is an optical technique and limited penetration (2 - 3 mm), its use is restricted to superficial tissues or those exposed through corridor surgery. The unique spectra of chemicals in tissue provide the potential to use hyperspectral imaging to image chemical content and from this provide useful qualitative or quantitative information to the surgeon to assist in decision making during the surgery. Chemical imaging can be used to differentiate between different tissues based on differing chemical composition and associated differing absorption (e.g., white vs grey matter), determine tissue state (e.g., normal vs malignant), and determine tissue status and/or health (e.g., state of oxygenation). The difference in spectral scattering properties can, similar to absorption changes, be used to determine the properties of tissue based on changes in cellular structure with tissue type (e.g., fat vs nerve fiber) and state (e.g., changes in nuclear and overall cell size with pre and cancerous states). Lastly, as the acquired hyperspectral data set contains data acquired at a variety of wavelength, images at only selected wavelengths or wavelength ranges to improve the visualization of tissue (minima or maxima in absorption or scattering). For example, images at wavelengths where hemoglobin absorption is at a minimum, the absorption due to blood will be significantly reduced thus providing additional light for illumination.

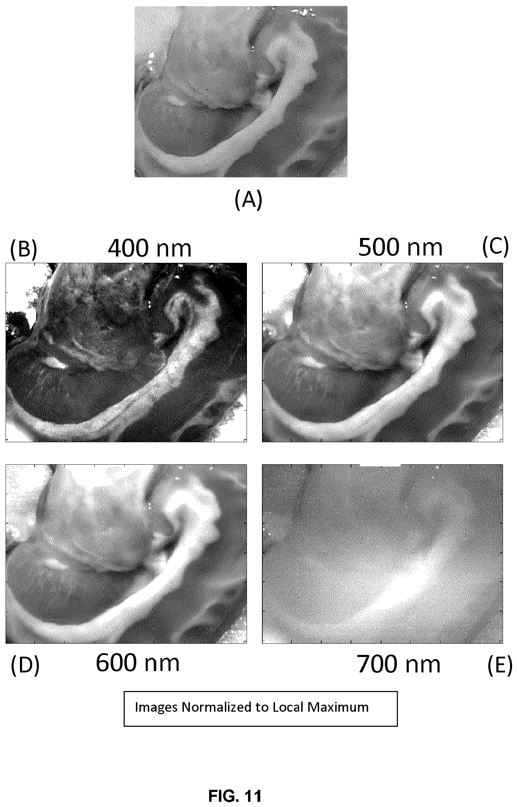

This advantage of imaging at specific wavelength bands is illustrated in **FIG. 11. FIG. 11** illustrates a standard color image (A) of a brain region (Corpus Callosum) that is also captured using four different wavelength bands centered at 400nm (B), 500nm (C), 600nm (D) and 700nm (E) and a bandwidth of 10nm each. It is evident that that 400nm filter band clearly illustrates tissue structures that are otherwise invisible in other wavelength bands.

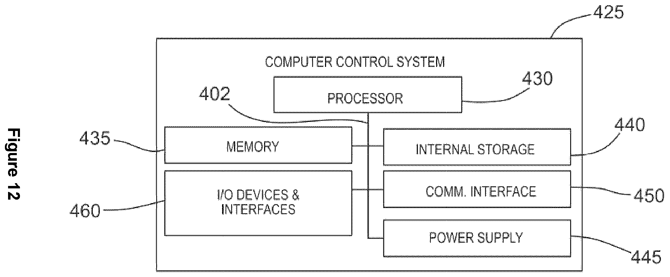

**FIG. 12** illustrates the key components of the computer system 1185 of **FIG. 9****.** **FIG. 12** provides an example, non-limiting implementation of computer control system **425,** which includes one or more processors **430** (for example, a CPU/microprocessor), bus **402,** memory **435,** which may include random access memory (RAM) and/or read only memory (ROM), one or more internal storage devices **440** (e.g. a hard disk drive, compact disk drive or internal flash memory), a power supply **445,** one more communications interfaces **450,** and various input/output devices and/or interfaces **460** such as a user interface for a clinician to provide various inputs, run simulations etc.

Although only one of each component is illustrated in **FIG. 12****,** any number of each component can be included computer control system **425.** For example, a computer typically contains a number of different data storage media. Furthermore, although bus **402** is depicted as a single connection between all of the components, it will be appreciated that the bus **402** may represent one or more circuits, devices or communication channels which link two or more of the components. For example, in personal computers, bus **402** often includes or is a motherboard.

In one embodiment, computer control system **425** may be, or include, a general purpose computer or any other hardware equivalents configured for operation in space. Computer control system **425** may also be implemented as one or more physical devices that are coupled to processor **430** through one of more communications channels or interfaces. For example, computer control system **425** can be implemented using application specific integrated circuits (ASIC). Alternatively, computer control system **425** can be implemented as a combination of hardware and software, where the software is loaded into the processor from the memory or over a network connection.

In another example embodiment, a vertical slit or a focal point may be imaged by the video scope using a confocal optical design that is commonly used in a microscope (not shown). The spot or slit may be then imaged on a photomultiplier to generate a very sensitive hyper-spectral imaging system. The focal point may be swept across the sample surface using a scanning mechanism. A commonly used scanning mechanism is a galvanometer mirror system.

The specific embodiments described above have been shown by way of example, and it should be understood that these embodiments may be susceptible to various modifications and alternative forms.

While the Applicant's teachings described herein are in conjunction with various embodiments for illustrative purposes, it is not intended that the applicant's teachings be limited to such embodiments. On the contrary, the applicant's teachings described and illustrated herein encompass various alternatives, modifications, and equivalents, without departing from the embodiments, the general scope of which is defined in the appended claims.

## Claims

1. A computer implemented method of intraoperatively confirming location of organized tissue structures in relation to a patient's skeletal structure, comprising:
acquiring preoperative image data of a patient's skeletal structure in a vicinity of an anatomical part;
acquiring intraoperative image data by scanning a selected region of tissue, in a vicinity of the skeletal structure in the anatomical part using Polarization Sensitive-Optical Coherence Tomography (PS-OCT);
identifying, from the intraoperative (PS-OCT) image data, regions of tissue exhibiting structural organization in the vicinity of the skeletal structure; and
using *a priori* known anatomical information about the regions of tissue exhibiting structural information for geometrically correlating and registering the intraoperative (PS-OCT) image data with the preoperative image data of the skeletal structure in the vicinity of the anatomical part.

2. The method according to claim 1, wherein the pre-operative image data of the skeletal structure in a vicinity of an anatomical part is acquired using any one of computed tomography (CT), magnetic resonance imaging (MRI) and optical coherence tomography (OCT).

3. The method according to claim 2, wherein the magnetic resonance imaging is T1 magnetic resonance imaging (T1 MRI).

4. The method according to any one of claims 1 to 3, wherein the tissue exhibiting structural organization includes, ligaments, tendons, muscle, cartilage, connective membranes, nerves, retina, blood vessel walls, some bone structures, trachea, esophagus, tongue, teeth and other connective tissues.

5. The method according to any one of claims 1 to 4, wherein the *a priori* known anatomical information about the regions of tissue exhibiting structural information include attachment points of tissue exhibiting structural information to the skeletal structure relative to landmark positions on the skeletal structure.

6. A computer implemented system for intraoperatively confirming location of organized tissue structures in relation to a patient's skeletal structure, comprising:
a Polarization Sensitive-Optical Coherence Tomography (PS-OCT) apparatus configured to scan a selected region of tissue to acquire intraoperative image data of the selected region of tissue;
a computer processor (430) having a memory storage (435), said Polarization Sensitive-Optical Coherence Tomography apparatus being connected to the computer processor, said memory storage having stored therein preoperative image data of a patient's skeletal structure in a vicinity of an anatomical part, said memory storage having stored therein a *priori* known anatomical information about the regions of tissue exhibiting structural information;
said computer processor being programmed with instructions to
a) identify, from the intraoperative (PS-OCT) image data, regions of tissue exhibiting structural organization in the vicinity of the skeletal structure; and
b) use the stored *priori* known anatomical information about the regions of tissue exhibiting structural information to geometrically correlate and register the intraoperative (PS-OCT) image data with the preoperative image data of the skeletal structure in the vicinity of the anatomical part.

## Patentansprüche

1. Ein computerimplementiertes Verfahren zur intraoperativen Bestätigung der Position organisierter Gewebestrukturen in Bezug auf die Skelettstruktur eines Patienten, umfassend:
Erfassen von präoperativen Bilddaten der Skelettstruktur eines Patienten in der Umgebung eines anatomischen Bereichs;
Erfassen intraoperativer Bilddaten durch Scannen eines ausgewählten Gewebebereichs in der Umgebung der Skelettstruktur in dem anatomischen Teil unter Verwendung der polarisationsempfindlich-optischen Kohärenztomographie (PS-OCT);
Identifizieren von Geweberegionen, die eine strukturelle Organisation in der Umgebung der Skelettstruktur aufweisen, auf Basis der intraoperativen (PS-OCT) Bilddaten; und
Verwendung von a *priori* bekannten anatomischen Informationen über die Gewebebereiche, die strukturelle Informationen aufweisen, um die intraoperativen (PS-OCT) Bilddaten mit den präoperativen Bilddaten der Skelettstruktur in der Umgebung des anatomischen Bereichs geometrisch zu korrelieren und aufzuzeichnen.

2. Verfahren nach Anspruch 1, wobei die präoperativen Bilddaten der Skelettstruktur in der Umgebung eines anatomischen Bereichs unter Verwendung von Computertomographie (CT), Magnetresonanztomographie (MRT) und optischer Kohärenztomographie (OCT) erfasst werden

3. Verfahren nach Anspruch 2, wobei die Magnetresonanztomographie eine T1-Magnetresonanztomographie (T1-MRT) ist.

4. Verfahren nach einem der Ansprüche 1 bis 3, wobei das Gewebe, das eine strukturelle Organisation aufweist, Bänder, Sehnen, Muskeln, Knorpel, Bindemembranen, Nerven, Netzhaut, Blutgefäßwände, einige Knochenstrukturen, Luftröhre, Speiseröhre, Zunge, Zähne und andere Bindegewebe umfasst

5. Verfahren nach einem der Ansprüche 1 bis 4, wobei die *a priori* bekannten anatomischen Informationen über die Bereiche des Gewebes, die strukturelle Informationen aufweisen, Befestigungspunkte von Gewebe umfassen, die strukturelle Informationen an der Skelettstruktur relativ zu Landmarkenpositionen auf der Skelettstruktur aufweisen.

6. Ein computerimplementiertes System zur intraoperativen Bestätigung der Position organisierter Gewebestrukturen in Bezug auf die Skelettstruktur eines Patienten, umfassend:
eine polarisationsempfindlich-optische Kohärenztomographie (PS-OCT) -Vorrichtung, die konfiguriert ist, um einen ausgewählten Gewebebereich abzutasten, um intraoperative Bilddaten des ausgewählten Gewebebereichs zu erfassen;
einen Computerprozessor (430) mit einem Speicher (435), wobei die polarisationsempfindlich-optische Kohärenztomographievorrichtung mit dem Computerprozessor verbunden ist, wobei der Speicher die präoperative Bilddaten der Skelettstruktur eines Patienten in der Umgebung eines anatomischen Bereichs gespeichert hat, wobei der Speicher *a priori* bekannte anatomische Informationen über die Gewebebereiche gespeichert hat, die strukturelle Informationen aufweisen;
wobei der Computerprozessor mit Anweisungen programmiert wird, um
a) aus den intraoperativen (PS-OCT) Bilddaten Bereiche des Gewebes zu identifizieren, die eine strukturelle Organisation in der Umgebung der Skelettstruktur aufweisen; und
b) Verwendung der gespeicherten *a priori* bekannten anatomischen Informationen über die Gewebebereiche, die strukturelle Informationen aufweisen, um die intraoperativen (PS-OCT) Bilddaten mit den präoperativen Bilddaten der Skelettstruktur in der Umgebung des anatomischen Bereichs geometrisch zu korrelieren und aufzuzeichnen.

## Revendications

1. Procédé mis en œuvre par ordinateur pour confirmer pendant l'opération la position de structures de tissus organisés par rapport à la structure squelettique d'un patient, comprenant :
l'acquisition de données d'images préopératoires de la structure squelettique d'un patient à proximité d'une région anatomique ;
l'acquisition de données d'image pendant l'opération en balayant une région de tissu sélectionnée à proximité de la structure squelettique dans la partie anatomique en utilisant la tomographie par cohérence optique sensible à la polarisation (PS-OCT) ;
identifier les régions de tissu présentant une organisation structurelle à proximité de la structure squelettique sur la base des données d'image pendant l'opération (PS-OCT) ; et
l'utilisation d'informations anatomiques connues *a priori* sur les régions de tissu présentant des informations structurelles pour corréler géométriquement et enregistrer les données d'image pendant l'opération (PS-OCT) avec les données d'image préopératoires de la structure squelettique entourant la région anatomique.

2. Le procédé de la revendication 1, dans lequel les données d'image pendant l'opération de la structure squelettique au voisinage d'une région anatomique sont acquises en utilisant la tomographie par ordinateur (CT), l'imagerie par résonance magnétique (IRM) et la tomographie par cohérence optique (OCT).

3. Le procédé de la revendication 2, dans lequel l'imagerie par résonance magnétique est une imagerie par résonance magnétique T1 (IRM T1).

4. Le procédé de l'une quelconque des revendications 1 à 3, dans lequel le tissu présentant une organisation structurelle comprend des ligaments, des tendons, des muscles, du cartilage, des membranes de liaison, des nerfs, la rétine, des parois de vaisseaux sanguins, certaines structures osseuses, la trachée, l'œsophage, la langue, les dents et d'autres tissus conjonctifs.

5. Le procédé de l'une quelconque des revendications 1 à 4, dans lequel les informations anatomiques connues *a priori* concernant les régions du tissu ayant des informations structurelles comprennent des points de fixation du tissu ayant des informations structurelles à la structure du squelette par rapport à des positions de repère sur la structure du squelette.

6. Un système mis en oeuvre par ordinateur pour confirmer pendant l'opération la position de structures de tissus organisés par rapport à la structure squelettique d'un patient, comprenant :
un dispositif de tomographie par cohérence optique sensible à la polarisation (PS-OCT) configuré pour balayer une région de tissu sélectionnée pour acquérir des données d'image pendant l'opération de la région de tissu sélectionnée ;
un processeur informatique (430) ayant une mémoire (435), le dispositif de tomographie par cohérence optique sensible à la polarisation étant couplé au processeur informatique, la mémoire contenant des données d'images préopératoires de la structure du squelette d'un patient au voisinage d'une région anatomique, la mémoire contenant des informations anatomiques connues *a priori* sur les régions de tissu ayant des informations structurelles;
dans lequel le processeur de l'ordinateur est programmé avec des instructions pour
a) identifier à partir des données d'image pendant l'opération (PS-OCT) des zones du tissu ayant une organisation structurelle à proximité de la structure squelettique ; et
b) utiliser les informations anatomiques connues *a priori* stockées concernant les zones de tissu présentant des informations structurelles pour corréler géométriquement et enregistrer les données d'image pendant l'opération (PS-OCT) avec les données d'image préopératoires de la structure du squelette à proximité de la zone anatomique.
